# EUROPEAN PATENT SPECIFICATION

(11) **EP 4 583 699 B1**
(45) Date of publication and mention of the grant of the patent: **05.08.2026**
(21) Application number: 23767878.4
(22) Date of filing: 06.09.2023
(51) Int. Cl.: A01K 1/00, B01D 53/74, B01D 53/75, B01D 53/86, A61L 9/14, A61L 9/015, A01C 3/02, A61L 9/20, A01C 3/00, B01D 53/88, B01D 53/64, B01D 53/58, B01D 53/62

(54) **EQUIPMENT FOR DECONTAMINATION OF AIR EVAPORATED FROM CONTAMINATED SLUDGE**
VORRICHTUNG ZUR DEKONTAMINATION VON AUS KONTAMINIERTEM SCHLAMM VERDAMPFTER LUFT
ÉQUIPEMENT DE DÉCONTAMINATION D'AIR ÉVAPORÉ À PARTIR DE BOUES CONTAMINÉES

(30) Priority: 07.09.2022 EP 22382833
(43) Date of publication of application: 16.07.2025
(73) Proprietor: Motors I Ventiladors, S.L., 08211 Castellar del Vallés (ES); Airtecnics Export SL, 08211 Castellar Del Vallés (ES)
(72) Inventor: MONAGAS ASENSIO, Pedro, 08211 CASTELLAR DEL VALLÈS (ES)
(74) Representative: ZBM Patents - Zea, Barlocci & Markvardsen
(86) International application number: PCT/EP2023/074486
(87) International publication number: WO 2024/052419

(56) References cited:
- ES-U- 1 291 640
- JP-A- 2019 195 056
- KR-A- 20080 013 831
- KR-B1- 101 787 402
- US-A1- 2007 212 273

## Description

### Field of the Invention

The present invention refers to an equipment for decontamination of air evaporated from contaminated sludge that is contained in an enclosure. In particular, it refers to an equipment for purification of air contaminated with emissions captured in farms, treatment centers or deposits of liquid or semiliquid manure and similar sludges or substances. It allows the elimination of odours, the reduction of pollution and the recovery of recoverable substances.

It may be applied in the field of livestock breeding, especially pigs.

The present invention also refers to an enclosure for contaminated sludge comprising said equipment for decontamination.

In the present disclosure, the term "sludge" is understood as sludge of animal origin, i.e. liquid manure. Also, it may be a sludge of mineral origin, for example brine, i.e. water with a dissolved salt concentration of more than 5 per cent, or sludge of vegetable origin, for example olive mill wastewater ("alpechin").

### State of the Art

Farms, especially pig farms, generate a whole series of dry and liquid waste (liquid manure) that must be treated. The main problem is liquid or semi-liquid waste, which is especially rich in nitrogenous compounds and whose uncontrolled discharge can cause the eutrophication of water masses and the contamination of aquifers. Therefore, this liquid waste has to be effectively controlled.

In the case of air purification from farms or liquid manure deposits, volatile substances are highly polluting and require a deeper treatment. One of the main polluting agents is ammonia.

Urea breaks down into water and ammonia. This reaction reaches its maximum ammonia emission at ambient pressure between 25 and 45°C. On the other hand, there are known farms with underfloor heating that raises the temperature of the waste and heated tanks, so that it can be close to the temperature with the highest ammonia emission. Therefore, the air present in farms and warehouses can have a high content of ammonia that has to be captured.

In the state of the art, ventilation equipment is known that offers photocatalytic installations, treatment plants, etc. although no equipment specially prepared for farms or facilities with liquid manure is known.

Patent ES2864155 is considered the state of the art closest to the present invention.

This patent discloses a ventilation and purification device that uses a photocatalytic substrate connected to ground or a direct current, an ozone emitter and a head that generates hydroxyl radicals. Other relevant documents are US 2007/212273 A1, KR 2008 0013831 A, JP 2019 195056 A, KR 101 787 402 B1 and ES 1 291 640 U.

The applicant is not aware of any equipment that allows similar advantages to be obtained or that can be considered similar to the invention.

### Description of the invention

The present invention relates to an equipment for decontamination of air evaporated from contaminated sludge, according to the claims. Its different embodiments solve the problems of the state of the art and provide noteworthy advantages.

According to one aspect, the present invention refers to an equipment for decontamination of air evaporated from contaminated sludge that is contained in an enclosure, the equipment comprising:
- a purifier with an air inlet connectable to the enclosure and an air outlet connectable to the enclosure, such that air from the enclosure recirculates through the purifier, the purifier comprising:
   - one or more filters;
   - a condenser arranged downstream of at least one of filters, the condenser being configured to remove the humidity from the air;
   - a water outlet for water removed from the air by the condenser;
   - a hydroxyl generator arranged downstream of the condenser, the hydroxyl generator comprising a tank of hydrogen peroxide with a nebulizer, and an ozone source;
   - a photocatalysis system arranged downstream of the hydroxyl generator; and
   - an impulsion unit for driving a flow of air from the enclosure along the purifier from the air inlet to the air outlet, through the filters, condenser, hydroxyl generator and photocatalysis system.

In the present disclosure the "air from the enclosure recirculates" means the air contained inside the enclosure, which evaporates from the liquid sludge, flows in closed circuit, in this case, through the purifier and the enclosure, such that over time and in several cycles the air passes through the purifier and is progressively decontaminated.

By virtue to the combination of filtration, generation of radical hydroxyls and photocatalysis, the equipment of the present invention allows a suitable decontamination and mineralization of the air evaporated from the contaminated sludge that is contained in the enclosure.

Thanks to the Advanced Oxidation Processes (AOPs) and specifically generating OH use of hydroxyl radicals, it allows the decomposition of volatile organic compounds (VOC) mainly into carbon dioxide. Likewise, it is highly effective against bacteria and effective against viruses. It also degrades the ammonia content into water and nitrogen, thus obtaining a mineralization of the air. Moreover, odours are also eliminated.

Furthermore, it oxidizes carbon monoxide and part of the carbon dioxide becomes carbonates and bicarbonates and water.

Regarding the hydroxyl generator, it is preferably that the tank of hydrogen peroxide and the ozone source be arranged close together so that the synergy between them favours a reaction between hydrogen peroxide and ozone that enhances the presence of hydroxyl radicals.

Furthermore, it is important that the hydroxyl generator is placed upstream the photocatalysis system, to ensure that the oxidation effect of the hydroxyl radicals is concentrated in said area.

The equipment of the present invention also allows the recovery of recoverable substances. On the one hand, water obtained from the sludge may be collected for further uses, e.g., for irrigation, or even for consumption (after treatment of ammoniacal nitrogen). On the other hand, dry matter of the sludge may be collected, e.g. for using as a fertilizer (in case of the sludge is liquid manure).

Experimental tests using the equipment of the present invention indicated that it may be obtained, e.g. 80 g of dry organic matter from 1 liter of slurry, and e.g. 70 liters of water from 100 liters of slurry.

The use of the condenser allows to reduce the humidity of the air, before it passes through the one or more filters, the hydroxyl generator and the photocatalysis system. It is noted that this dry and filtered-clean air has a higher efficiency and capacity of reacting in chain with the amount of molecules generated of hydroxyl radicals.

According to one embodiment of the present invention, the equipment further may comprise a heating circuit for heating the air in the enclosure, the heating circuit being provided with an air inlet connectable to the enclosure and an air outlet connectable to the enclosure, such that air from the enclosure recirculates through the heating circuit.

The heating circuit allows the temperature inside the enclosure to be raised, producing an increase in evaporation and the rise of gases (odours, volatile compounds, carbon dioxide...) that are captured by the purifier. In this way, the liquid waste is dried or reduces its water content, achieving the recovery of the solid organic matter that is part of the liquid waste.

In a preferred example, the heating circuit may comprise:
- a condenser arranged downstream of the air inlet, the condenser being configured for eliminating the humidity of the air;
- a solar panel heat exchanger for heating the flow of air, arranged downstream of the condenser; and
- an impulsion unit for driving a flow of air from the air inlet to the air outlet of the heating circuit.

According to one embodiment, the equipment further comprises an extractor of air from the enclosure to the environment. The extractor may be used when it is necessary to evacuate the air, to reduce odours, allow personnel to enter the enclosure or else. The extractor may comprise a filtering unit and a photocatalytic unit in contact with the airflow.

According to another aspect, the present invention also provides an enclosure for contaminated sludge. In one example, the enclosure may be a greenhouse, e.g. with transparent walls, to increase the interior temperature, when it is not necessary to maintain interior habitability-conditions.

Further embodiments of the invention will be disclosed in the following.

The liquid manure emission purification equipment is incorporated in an enclosure that includes liquid waste or liquid manure. It includes an air purification circuit, with return to the enclosure itself. This purification circuit incorporates a purifier that contains:
- One or more filters.
- A hydroxyl generator. The hydroxyl is generated from hydrogen peroxide, in which case it has a tank of hydrogen peroxide connected to a evaporation case by airflow
- An ozone source.
- A photocatalysis system. This system is usually based on materials such as titanium dioxide, illuminated by UV-C light and/or connected to a direct current.

The purification circuit comprises a condenser attached to the filters.

As explained above, thanks to the Advanced Oxidation Processes (AOPs) and specifically generating OH use of hydroxyl radicals, it allows the decomposition of volatile organic compounds (VOC) mainly into carbon dioxide. Likewise, it is highly effective against bacteria and effective against viruses. It also degrades the ammonia content.

Furthermore, it oxidizes carbon monoxide and part of the carbon dioxide becomes carbonates and bicarbonates.

The invention may also comprise a heating circuit for the air in the enclosure. This heating circuit can include a geothermal heat source, resistors, aerothermal... but it is preferably solar.

Thus, in the most preferred embodiment, the heating circuit comprises a condenser unit that removes the humidity prior to a solar panel for heating the air. In other words, the enclosure's own air passes through a panel for heating. If, in addition, the panel includes a photocatalytic coating in contact with this air, part of the oxidation treatment is carried out in this heating circuit.

If it is necessary to evacuate the air, to reduce odours, allow personnel to enter the enclosure or else, an extractor fan of the air from the enclosure to the environment may be added. To avoid dispersion of the troublesome substances, it is desirable that the extractor comprises one or more filter units and a photocatalytic unit.

Other variants will be shown in the rest of the specification.

### Description of the Drawings

For a better understanding of the invention, the following figures are included.
Figure 1 is a schematic diagram of an embodiment of the equipment of the invention;
Figure 2 and Figure 3 are, respectively, a schematic elevation view and a schematic plan view of a purifier according to one embodiment of the invention;
Figure 4 is a simplified diagram of a further embodiment of the equipment of the invention; and
Figure 5 is a schematic cross-section view of a Z-shaped filter according to one embodiment of the invention.

### Description of a preferred embodiment of the invention

Next, an embodiment of the invention is briefly described, as an illustrative and nonlimiting example of the invention.

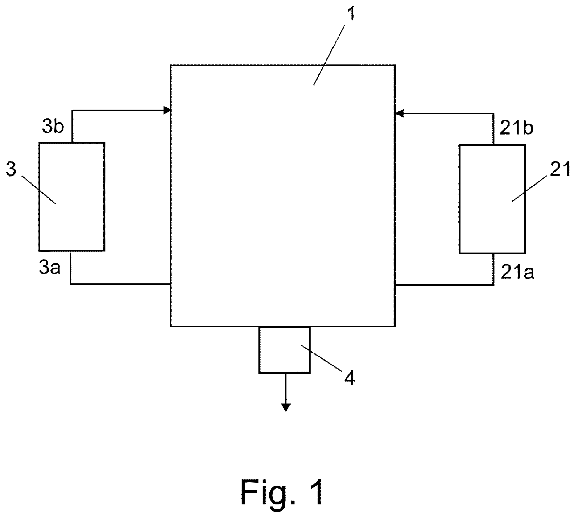

Figure 1 shows schematically an equipment for decontamination of air evaporated from contaminated sludge that is contained in an enclosure 1, according to an embodiment of the present invention. The equipment comprising:
- a purifier 21 with an air inlet 21a connectable to the enclosure 1 and an air outlet 21b connectable to the enclosure 1, such that air from the enclosure 1 recirculates through the purifier;
- a heating circuit 3 for heating the air in the enclosure 1, the heating circuit 3 being provided with an air inlet 3a connectable to the enclosure 1 and an air outlet 3b connectable to the enclosure 1, such that air from the enclosure 1 recirculates through the heating circuit 3; and
- an extractor 4 for extracting the air from the enclosure 1 to the environment.

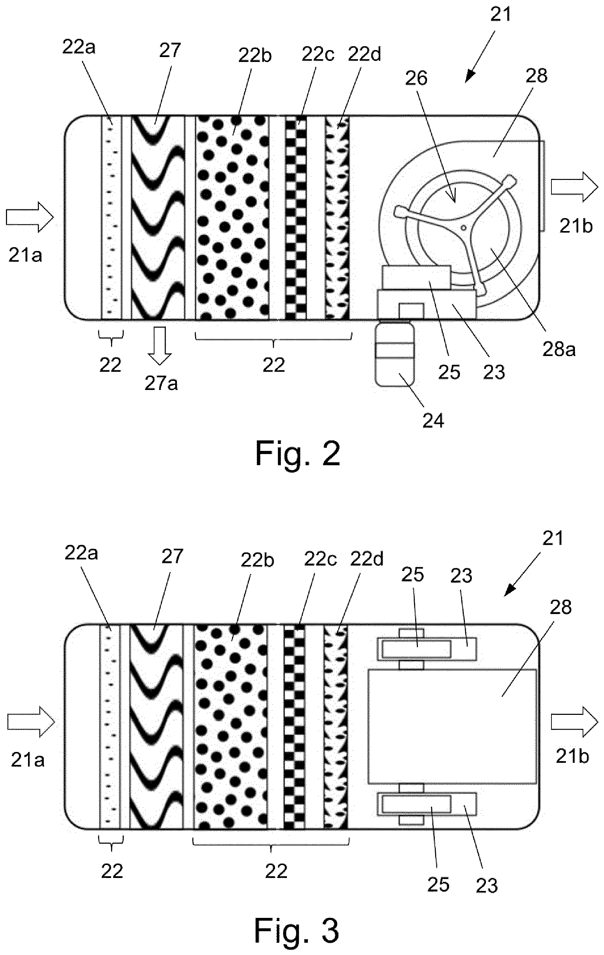

Figures 2 and 3 show, respectively, a schematic elevation view and a schematic plan view of a purifier 21 according to an embodiment of the present invention. According to this example, the purifier 21 comprises:
- one or more filters 22 as will be described in more detail below;
- a condenser 27 arranged downstream of at least one of filters 22, the condenser 27 being configured to remove the humidity from the air;
- a water outlet 27a for water removed from the air by the condenser 27;
- a hydroxyl generator 23 arranged downstream of the condenser 27, the hydroxyl generator 23 comprising a tank 24 of hydrogen peroxide with a nebulizer, and an ozone source 25;
- a photocatalysis system 26 arranged downstream of the hydroxyl generator 23; and
- an impulsion unit 28 for driving a flow of air from the enclosure 1 along the purifier 21 from the air inlet 21a to the air outlet 21b, through the filters 22, condenser 27, hydroxyl generator 23 and photocatalysis system 26.

Thanks to the purifier 21, as disclosed above, the decomposition of ammonia into water and nitrogen takes place as well as the oxidation of volatile organic compounds and the destruction of possible viruses, molds and bacteria. Furthermore, CO2 is becoming by effect of the hydroxyl radicals into carbonate, bicarbonate and water.

The condenser 27 may be, for example, a Peltier cell, a water battery or other cooling systems capable to reduce the humidity from the air.

Furthermore, the water outlet 27a allows the evacuation of water removed from the air by the condenser 27. This water may be collected e.g. into a tank (not shown) for later uses, for example for irrigation, or even for consumption (after treatment of ammoniacal nitrogen).

As can be seen in the embodiment shown in figures 2 and 3, the set of filters 22 may comprise several filters depending on the gases to be decontaminated contained in an enclosure. For example, in this embodiment the set of filters 22 comprises:
- a particulate filter 22a, preferably a G4 filter, arranged upstream the condenser 27, wherein the particulate filter 22a allows particulate matter suspended in the contaminated air to be retained before the air reaches the condenser 27;
- a drop separator filter 22b arranged downstream the condenser 27, and arranged before the rest of filters so that humidity is captured in order to not affect them;
- a mineral filter 22c, for example from zeolites, arranged downstream the drop separator 22b, wherein the mineral filter 22c allows to retain ammonium and other gases; and
- a vegetable filter 22d arranged downstream the mineral filter 22c.

The vegetable filter 22d may comprise a vegetal body with a low pH (acid), preferably with a carboxylic acid. This acid may be obtained from pectin.

Heavy metals (mainly Cu, Zn and K) and other components may also be eliminated with a bio-adsorbent as citric-fruit peelings, mainly orange and lemon peelings. These peelings are a waste product from several food industry processes.

Urea also breaks at those temperatures (25-40°C) into water, ammonia, and carbon dioxide. The ammonia can be filtered with a bio-adsorbent. An example of bio-adsorbent is citric-fruit peelings. These peelings may be placed in any vegetal filter of the equipment of the present invention.

As explained above, the condenser 27 allows to reduce the humidity of the air, before it passes through the rest of filters, the hydroxyl generator 23 and the photocatalysis system 26. It is noted that this dry and filtered-clean air has a higher efficiency and capacity of reacting in chain with the amount of molecules generated of hydroxyl radicals.

As shown in figures 2 and 3, the hydroxyl generator 23 is arranged downstream of the condenser 27. Furthermore, in this embodiment, the equipment is provided with two hydroxyl generators 23, each one comprising a tank 24 of hydrogen peroxide with a nebulizer, to be atomized in the air flow, and an ozone source 25.

Regarding the hydroxyl generator 23, it is preferably that the tank 24 of hydrogen peroxide and the ozone source 25 be arranged close together so that the synergy between them favours a reaction between hydrogen peroxide and ozone that enhances the presence of hydroxyl radicals.

Furthermore, it is important that the hydroxyl generator 23 is placed upstream the photocatalysis system 26, to ensure that the oxidation effect of the hydroxyl radicals is concentrated in said area.

The photocatalysis system 26 may comprise a photocatalytic coating material in contact with the flow of air. The photocatalytic coating material is made of materials with electro-photocatalysis properties that emit radiation at the ultraviolet frequency. Titanium oxide (e.g. titanium dioxide), a metal nitride, strontium titanate and other metal oxides with the ability to absorb in the visible spectrum, modified with transition metal dimers and trimers, are mentioned as examples. The photocatalytic coating material is connected to a conductor. The conductor may form part of a direct-current circuit or be connected to earth, in which case the coating is illuminated by at least one UV light emitted, for example, by one or more LEDs. This connection provides the photocatalyst with germicidal properties. In the case wherein the conductor is connected to a direct current, a UV light source can also be added.

Furthermore, the photocatalytic coating material may be applied on surfaces of the impulsion unit 28 that are in contact with the flow of air.

The impulsion unit 28 may be for example, a centrifugal fan, a pump or a compressor.

In the embodiment shown in figures 2 and 3, the impulsion unit is a centrifugal fan 28 placed adjacent to the air outlet 2b. The centrifugal fan 28 comprises a housing and a drum-shaped impeller 28a with fan blades mounted around a hub. Furthermore, in this embodiment, the photocatalytic coating material may be applied on the fan blades and/or on the internal walls of the housing of the centrifugal fan 28, such that a turbulent regime is generated that maximises contact of the photocatalytic coating material with the flow of air, thus increasing the oxidation effect. Moreover, in this embodiment, the UV light emitter (not shown), e.g. a led lamp, is arranged within the drum-shaped impeller to illuminate the photocatalytic coating material.

As disclosed above, the equipment of the present disclosure further comprises a heating circuit 3 (see figure 1) for heating the air in the enclosure 1, the heating circuit 3 being provided with an air inlet 3a connectable to the enclosure 1 and an air outlet 3b connectable to the enclosure 1, such that air from the enclosure 1 recirculates through the heating circuit 3.

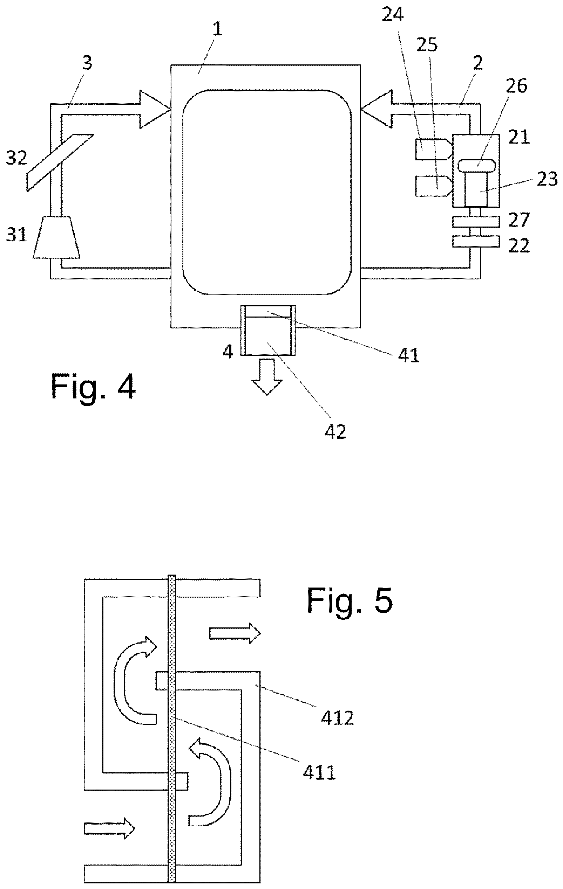

According to an embodiment (see figure 4), the heating circuit 3 comprises:
- a condenser 31 arranged downstream of the air inlet 3a, the condenser 31 being configured for eliminating the humidity of the air;
- a solar panel heat exchanger 32 for heating the flow of air, arranged downstream of the condenser 31; and
- an impulsion unit (not shown) for driving a flow of air from the air inlet 3a to the air outlet 3b of the heating circuit 3. For example, the impulsion unit may be placed between the condenser 31 and the solar panel heat exchanger 32.

In the present disclosure, the "solar panel heat exchanger" is understood as a heat exchanger configured as a panel with a serpentine circuit for the flow of air, and exposed to sunlight.

In operation, the heating circuit 3 allows the temperature inside the enclosure 1 to be raised, producing an increase in evaporation and the rise of gases (odours, volatile compounds, carbon dioxide...) that are captured by the purifier 21. In this way, the liquid waste is dried or reduces its water content, achieving the recovery of the solid organic matter that is part of the liquid waste. Other drying equipment (thermal or filtered) can be incorporated to finish removing the water from the liquid waste.

The solar panel heat exchanger 32 allows the passage of radiation to dry air from the enclosure 1, thus producing decomposition by photocatalysis. In order to increase said decomposition, the solar panel heat exchanger 32 may comprise a photocatalytic
coating material, such as titanium dioxide, in contact with the airflow, that is illuminated by sunlight or an UV light. This photocatalytic coating material is preferably earthed. As explained above, photocatalysis helps oxidize compounds present in the air. The photocatalytic coating material may be deposited on the walls of the heating circuit 3 with a sol-gel process, spattering, or vapor phase deposition.

The heating circuit 3 may also comprise filters, for example, it may comprise a vegetal body with a low pH (acid), preferably with a carboxylic acid. This acid may be obtained from pectin.

As disclosed above, the equipment of the present disclosure further comprises an extractor 4 for extracting the air from the enclosure 1 to the environment (see figure 1). The use of the extractor 4 allows obtaining the reduction of ammonia, particulate matter and VOC emissions into the environment.

According to an embodiment (see figure 4), the extractor 4 may comprise a filtering unit 41 and a photocatalytic unit 42 in contact with the airflow. For example, the filter unit 41 may be provided with a particulate filter, a carbon filter and a Z-shaped filter (see figure 2). A photocatalytic unit 42, such as the one disclosed in ES2864155, complements the purification prior to release into the environment.

The Z-shaped filter shown in figure 5 comprises a filter body 411 that is placed three times in the airflow, as shown with the arrows. A housing 412 ensures that the flow crosses three times the filter body 411. The filter body 411 may comprise a bio-adsorbent as, for example, citric-fruit peelings.

According to an embodiment, the enclosure 1 may be a greenhouse, e.g. with transparent walls, to increase the interior temperature, when it is not necessary to maintain interior habitability-conditions. In other examples, the enclosure may be a stable, a covered liquid manure tank, etc.

Figures 4 and 5 show further embodiment as explained in the following.

The device is incorporated into an enclosure (1), such as a stable, a covered liquid manure tank... The enclosure (1) has at least one ventilation device that captures the air inside it for purification. The enclosure (1) will not normally be hermetic, but it can be. Likewise, it is desirable that the enclosure (1) be a greenhouse, with transparent walls, to increase the interior temperature, when it is not necessary to maintain interior habitability-conditions.

The ventilation equipment has several components. A first component is a purification circuit (2) that takes and returns the air to the enclosure (1). This purification circuit (2) is based on a purifier (21) that contains one or more filters (22) and a hydroxyl generator (23). The hydroxyl generator (23) starts from a hydrogen peroxide tank (24) with a nebulizer, preferably consisting in evaporation by airflow and an ozone source (25). A photocatalysis system (26) is also incorporated into the purifier (21), for instance in a fan or turbine.

The filters (22) can be a mineral (zeolite) filter, a drop filter... and are accompanied by a condenser (27) to reduce humidity. The condenser (27) can be a Peltier cell, a water battery or other cooling systems. If used, the drop filter is placed before the rest of the filters (22) so the humidity is captured and does not affect them (figure 1).

In this purifier (21), the decomposition of ammonia into water and nitrogen takes place as well as the oxidation of volatile organic compounds and the destruction of possible viruses, molds and bacteria. CO2 is becoming by the hydroxyl radicals into carbonate, bicarbonate and water.

A heating circuit (3) allows for raising the temperature of the air in the enclosure (1).

The most preferred way is using solar radiation, with a condenser unit (31) that eliminates the humidity prior to a solar panel (32). The solar panel (32) being a heat exchanger. The panel (32) allows the passage of radiation to dry air from the enclosure (1), thus producing decomposition by photocatalysis. This decomposition may be increased by using a photocatalytic coating such as titanium dioxide that is illuminated by sunlight or an UV light. This coating is preferably earthed. Photocatalysis helps oxidize compounds present in the air. The photocatalytic product may be deposited on the walls of the heating circuit (3) with a sol-gel process, spattering, or vapor phase deposition.

The filters in any unit (2,3) may comprise a vegetal body with a low pH (acid), preferably with a carboxylic acid. This acid may be obtained from pectin.

The enclosure (1) may also incorporate an extractor (4), which allows the air to be sent outside. This extractor (4) comprises a filter unit (41) with a particulate filter, a carbon filter and a Z-shaped filter (figure 5). A photocatalytic unit (42), such as the one disclosed in ES2864155, complements the purification prior to release into the environment.

The Z-shaped filter shown in figure 2 comprises a filter body (411) that is placed three times in the airflow, as shown with the arrows. A housing (412) ensures that the flow crosses three times the filter body (411).

In operation, the heating circuit (3) allows the temperature inside the enclosure (1) to be raised, producing an increase in evaporation and the rise of gases (odours, volatile compounds, carbon dioxide...) that are captured by the purifying circuit (2). In this way, the liquid waste is dried or reduces its water content, achieving the recovery of the solid organic matter that is part of the liquid waste. Other drying equipment (thermal or filtered) can be incorporated to finish removing the water from the liquid waste.

All circuits (2,3) may require impulsion units (fans, compressors...),

Heavy metals (mainly Cu, Zn and K) and other components may also be eliminated with a bio-adsorbent as citric-fruit peelings, mainly orange and lemon peelings. These peelings are a waste product from several food industry processes.

Urea also breaks at those temperatures (25-40°C) into water, ammonia, and carbon dioxide. The ammonia can be filtered with a bio-adsorbent. An example of bio-adsorbent is citric-fruit peelings. These peelings may be placed in any filter, for instance in the filter body (411) of the Z-shaped filter.

The peelings need to be cleaned in order to eliminate wax or other surface treatments. They are then dried and grinded to a particle size of 0,5-3 mm. Pectin is removed and can be used with other vegetable bodies to form filter bodies (411) or any filter. First with an acid solution (e.g. HCI) at approximately 90°C, during at least one hour. Pectin can also be extracted in an acid solution in an ultrasonic chamber (45 minutes at 37KHz) at room temperature. The acid solution also degrades carbohydrates and hemicellulose, specially xylan.

The acid treatment is repeated until no sugars or pectin is found in the solution (tested with Fehling method for reducing sugars). It is usually enough with two cycles.

Any excess acid is removed from the peelings, for instance with distilled water.

These peelings have been tested for heavy metal absorption, in this case Cu(ll) removal from CuSO4·5H2O solutions (5, 10, 30 and 100 mg/l of Cu2+). An atomic absorption spectrometer Varian AA24OFS is used for Cu(ll) determination. 0,5 g of lemon or orange peelings are placed in a test tube with 25 ml of the solution. The tube is gently agitated for 1 hour and the Cu(ll) concentration measured.

| Bio-adsorbent | Initial [Cu(II)] mg/l | Final [Cu(II)] mg/l | % Adsorbed |
|---|---|---|---|
| Orange peelings | 5 | 1,25 | 75 |
| Orange peelings | 10 | 1,15 | 88,5 |
| Orange peelings | 30 | 2,69 | 91 |
| Orange peelings | 100 | 3,64 | 96,4 |
| Lemon peelings | 5 | 0,78 | 84,4 |
| Lemon peelings | 10 | 0,82 | 91,8 |
| Lemon peelings | 30 | 1,64 | 94,5 |
| Lemon peelings | 100 | 2,22 | 97,8 |

Other vegetal bodies, as sawdust, pine bark... may be used as the filter body (411) or in any other filter, if they are treated with the pectin in the acidized water from the citric-fruit peelings, so they obtain the same properties. Those vegetal bodies treated with pectin will retain the ammonia and produce a nitrogen fertilizer rich with amines, that just needs a compost phase.

The scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. Equipment for decontamination of air evaporated from contaminated sludge that is contained in an enclosure (1), the equipment comprising:
- a purifier (21) with an air inlet (21a) connectable to the enclosure (1) and an air outlet (21b) connectable to the enclosure (1), such that air from the enclosure (1) recirculates through the purifier, the purifier (21) comprising:
- one or more filters (22);
- a condenser (27) arranged downstream of at least one of filters (22), the condenser (27) being configured to remove the humidity from the air;
- a water outlet (27a) for water removed from the air by the condenser (27);
- a hydroxyl generator (23) arranged downstream of the condenser (27), the hydroxyl generator (23) comprising a tank (24) of hydrogen peroxide with a nebulizer, and an ozone source (25);
- a photocatalysis system (26) arranged downstream of the hydroxyl generator (23); and
- an impulsion unit (28) for driving a flow of air from the enclosure (1) along the purifier (21) from the air inlet (21a) to the air outlet (21b), through the filters (22), condenser (27), hydroxyl generator (23) and photocatalysis system (26).

2. Equipment according to claim 1, wherein the photocatalysis system (26) comprises a photocatalytic coating material in contact with the flow of air.

3. Equipment according to claim 2, wherein the photocatalytic coating material is either connected to earth and illuminated by a UV light or connected to a direct current power source.

4. Equipment according to claim 2 or 3, wherein the photocatalytic coating material is applied on surfaces of the impulsion unit (28) that are in contact with the flow of air.

5. Equipment according to any of the preceding claims, wherein the condenser (27) is a water battery or a Peltier cell.

6. Equipment according to any of the preceding claims, wherein the one or more filters (22) comprises a particulate filter (22a), preferably a G4 filter, arranged upstream the condenser (27).

7. Equipment according to any of the preceding claims, wherein the one or more filters (22) comprises a drop separator filter (22b) arranged downstream the condenser (27).

8. Equipment according to any of the preceding claims, wherein the one or more filters (22) comprises a mineral filter (22c) arranged downstream the condenser (27).

9. Equipment according to any of the preceding claims, wherein the one or more filters (22) comprises a vegetable filter (22d) arranged downstream the condenser (27).

10. Equipment according to any of the preceding claims, wherein the equipment further comprises a heating circuit (3) for heating the air in the enclosure (1), the heating circuit (3) being provided with an air inlet (3a) connectable to the enclosure (1) and an air outlet (3b) connectable to the enclosure (1), such that air from the enclosure (1) recirculates through the heating circuit (3).

11. Equipment according to claim 10, wherein the heating circuit (3) comprises
- a condenser (31) arranged downstream of the air inlet (3a), the condenser (31) being configured for eliminating the humidity of the air;
- a solar panel heat exchanger (32) for heating the flow of air, arranged downstream of the condenser (31), wherein the heat exchanger (32) is configured as a panel with a serpentine circuit for the flow of air, and exposed to sunlight; and
- an impulsion unit for driving a flow of air from the air inlet (3a) to the air outlet (3b) of the heating circuit (3).

12. Equipment according to claim 11, wherein the solar panel heat exchanger (32) comprises a photocatalytic coating material in contact with the flow of air.

13. Equipment according to any of preceding claims, wherein the equipment further comprises an extractor (4) of air from the enclosure (1) to the environment.

14. An enclosure for contaminated sludge, comprising an equipment according to any of claims 1 to 13.

15. Enclosure according to claim 14, wherein the enclosure (1) is a greenhouse.

## Patentansprüche

1. Vorrichtung zur Dekontamination von Luft, die aus kontaminiertem Schlamm verdampft ist, welcher in einer Einhausung (1) enthalten ist, wobei die Vorrichtung umfasst:
- einen Reiniger (21) mit einem Lufteinlass (21a), der mit der Einhausung (1) verbindbar ist, und einem Luftauslass (21b), der mit der Einhausung (1) verbindbar ist, derart, dass Luft aus der Einhausung (1) durch den Reiniger rezirkuliert, wobei der Reiniger (21) umfasst:
- einen oder mehrere Filter (22);
- einen Kondensator (27), der stromabwärts von mindestens einem der Filter (22) angeordnet ist, wobei der Kondensator (27) dazu ausgebildet ist, die Feuchtigkeit aus der Luft zu entfernen;
- einen Wasserauslass (27a) für das durch den Kondensator (27) aus der Luft entfernte Wasser;
- einen Hydroxylgenerator (23), der stromabwärts des Kondensators (27) angeordnet ist, wobei der Hydroxylgenerator (23) einen Tank (24) mit Wasserstoffperoxid mit einem Vernebler und eine Ozonquelle (25) umfasst;
- ein Photokatalysesystem (26), das stromabwärts des Hydroxylgenerators (23) angeordnet ist; und
- eine Antriebseinheit (28) zum Antreiben eines Luftstroms aus der Einhausung (1) entlang des Reinigers (21), vom Lufteinlass (21a) zum Luftauslass (21b), durch die Filter (22), den Kondensator (27), den Hydroxylgenerator (23) und das Photokatalysesystem (26).

2. Vorrichtung nach Anspruch 1, wobei das Photokatalysesystem (26) ein photokatalytisches Beschichtungsmaterial in Kontakt mit dem Luftstrom umfasst.

3. Vorrichtung nach Anspruch 2, wobei das photokatalytische Beschichtungsmaterial entweder geerdet und durch UV-Licht beleuchtet ist oder mit einer Gleichstromquelle verbunden ist.

4. Vorrichtung nach Anspruch 2 oder 3, wobei das photokatalytische Beschichtungsmaterial auf Oberflächen der Antriebseinheit (28) aufgebracht ist, die in Kontakt mit dem Luftstrom stehen.

5. Vorrichtung nach einem der vorhergehenden Ansprüche, wobei der Kondensator (27) eine Wasserbatterie oder eine Peltier-Zelle ist.

6. Vorrichtung nach einem der vorhergehenden Ansprüche, wobei der eine oder die mehreren Filter (22) einen Partikelfilter (22a), vorzugsweise einen G4-Filter, umfassen, der stromaufwärts des Kondensators (27) angeordnet ist.

7. Vorrichtung nach einem der vorhergehenden Ansprüche, wobei der eine oder die mehreren Filter (22) einen Tropfenabscheiderfilter (22b) umfassen, der stromabwärts des Kondensators (27) angeordnet ist.

8. Vorrichtung nach einem der vorhergehenden Ansprüche, wobei der eine oder die mehreren Filter (22) einen Mineralfilter (22c) umfassen, der stromabwärts des Kondensators (27) angeordnet ist.

9. Vorrichtung nach einem der vorhergehenden Ansprüche, wobei der eine oder die mehreren Filter (22) einen Pflanzenfilter (22d) umfassen, der stromabwärts des Kondensators (27) angeordnet ist.

10. Vorrichtung nach einem der vorhergehenden Ansprüche, wobei die Vorrichtung ferner einen Heizkreislauf (3) zum Erwärmen der Luft in der Einhausung (1) umfasst, wobei der Heizkreislauf (3) mit einem Lufteinlass (3a), der mit der Einhausung (1) verbindbar ist, und einem Luftauslass (3b), der mit der Einhausung (1) verbindbar ist, versehen ist, derart, dass Luft aus der Einhausung (1) durch den Heizkreislauf (3) rezirkuliert.

11. Vorrichtung nach Anspruch 10, wobei der Heizkreislauf (3) umfasst:
- einen Kondensator (31), der stromabwärts des Lufteinlasses (3a) angeordnet ist, wobei der Kondensator (31) dazu ausgebildet ist, die Feuchtigkeit aus der Luft zu entfernen;
- einen Solarpaneel-Wärmetauscher (32) zum Erwärmen des Luftstroms, der stromabwärts des Kondensators (31) angeordnet ist, wobei der Wärmetauscher (32) als ein Paneel mit einem Serpentinenkreislauf für den Luftstrom ausgebildet und dem Sonnenlicht ausgesetzt ist; und
- eine Antriebseinheit zum Antreiben eines Luftstroms vom Lufteinlass (3a) zum Luftauslass (3b) des Heizkreislaufs (3).

12. Vorrichtung nach Anspruch 11, wobei der Solarpaneel-Wärmetauscher (32) ein photokatalytisches Beschichtungsmaterial in Kontakt mit dem Luftstrom umfasst.

13. Vorrichtung nach einem der vorhergehenden Ansprüche, wobei die Vorrichtung ferner einen Extraktor (4) für Luft aus der Einhausung (1) in die Umgebung umfasst.

14. Einhausung für kontaminierten Schlamm, umfassend eine Vorrichtung nach einem der Ansprüche 1 bis 13.

15. Einhausung nach Anspruch 14, wobei die Einhausung (1) ein Gewächshaus ist.

## Revendications

1. Équipement pour la décontamination d'air évaporé à partir de boues contaminées contenues dans une enceinte (1), l'équipement comprenant:
- un épurateur (21) avec une entrée d'air (21a) raccordable à l'enceinte (1) et une sortie d'air (21b) raccordable à l'enceinte (1), de telle sorte que l'air provenant de l'enceinte (1) recircule à travers l'épurateur, l'épurateur (21) comprenant :
- un ou plusieurs filtres (22);
- un condenseur (27) disposé en aval d'au moins un des filtres (22), le condenseur (27) étant configuré pour éliminer l'humidité de l'air;
- une sortie d'eau (27a) pour l'eau éliminée de l'air par le condenseur (27);
- un générateur d'hydroxyle (23) disposé en aval du condenseur (27), le générateur d'hydroxyle (23) comprenant un réservoir (24) de peroxyde d'hydrogène avec un nébuliseur, et une source d'ozone (25);
- un système de photocatalyse (26) disposé en aval du générateur d'hydroxyle (23); et
- une unité d'impulsion (28) pour entraîner un flux d'air provenant de l'enceinte (1) le long de l'épurateur (21), depuis l'entrée d'air (21a) jusqu'à la sortie d'air (21b), à travers les filtres (22), le condenseur (27), le générateur d'hydroxyle (23) et le système de photocatalyse (26).

2. Équipement selon la revendication 1, dans lequel le système de photocatalyse (26) comprend un matériau de revêtement photocatalytique en contact avec le flux d'air.

3. Équipement selon la revendication 2, dans lequel le matériau de revêtement photocatalytique est soit relié à la terre et éclairé par une lumière UV, soit relié à une source d'alimentation en courant continu.

4. Équipement selon la revendication 2 ou 3, dans lequel le matériau de revêtement photocatalytique est appliqué sur des surfaces de l'unité d'impulsion (28) qui sont en contact avec le flux d'air.

5. Équipement selon l'une quelconque des revendications précédentes, dans lequel le condenseur (27) est une batterie à eau ou une cellule Peltier.

6. Équipement selon l'une quelconque des revendications précédentes, dans lequel le ou les filtres (22) comprennent un filtre à particules (22a), de préférence un filtre G4, disposé en amont du condenseur (27).

7. Équipement selon l'une quelconque des revendications précédentes, dans lequel le ou les filtres (22) comprennent un filtre séparateur de gouttes (22b) disposé en aval du condenseur (27).

8. Équipement selon l'une quelconque des revendications précédentes, dans lequel le ou les filtres (22) comprennent un filtre minéral (22c) disposé en aval du condenseur (27).

9. Équipement selon l'une quelconque des revendications précédentes, dans lequel le ou les filtres (22) comprennent un filtre végétal (22d) disposé en aval du condenseur (27).

10. Équipement selon l'une quelconque des revendications précédentes, dans lequel l'équipement comprend en outre un circuit de chauffage (3) pour chauffer l'air dans l'enceinte (1), le circuit de chauffage (3) étant pourvu d'une entrée d'air (3a) raccordable à l'enceinte (1) et d'une sortie d'air (3b) raccordable à l'enceinte (1), de telle sorte que l'air provenant de l'enceinte (1) recircule à travers le circuit de chauffage (3).

11. Équipement selon la revendication 10, dans lequel le circuit de chauffage (3) comprend:
- un condenseur (31) disposé en aval de l'entrée d'air (3a), le condenseur (31) étant configuré pour éliminer l'humidité de l'air ;
- un échangeur de chaleur à panneau solaire (32) pour chauffer le flux d'air, disposé en aval du condenseur (31), dans lequel l'échangeur de chaleur (32) est configuré comme un panneau avec un circuit en serpentin pour le flux d'air, et exposé à la lumière du soleil ; et
- une unité d'impulsion pour entraîner un flux d'air depuis l'entrée d'air (3a) jusqu'à la sortie d'air (3b) du circuit de chauffage (3).

12. Équipement selon la revendication 11, dans lequel l'échangeur de chaleur à panneau solaire (32) comprend un matériau de revêtement photocatalytique en contact avec le flux d'air.

13. Équipement selon l'une quelconque des revendications précédentes, dans lequel l'équipement comprend en outre un extracteur (4) d'air de l'enceinte (1) vers l'environnement.

14. Enceinte pour boues contaminées, comprenant un équipement selon l'une quelconque des revendications 1 à 13.

15. Enceinte selon la revendication 14, dans laquelle l'enceinte (1) est une serre.
